# EUROPÄISCHE PATENTSCHRIFT

(11) **EP 0 912 172 B1**
(45) Veröffentlichungstag und Bekanntmachung des Hinweises auf die Patenterteilung: **06.09.2000**
(21) Anmeldenummer: 97919269.7
(22) Anmeldetag: 11.03.1997
(51) Int. Cl.: A61K 31/40

(54) **KOMBINATION AUS CIS-4-HYDROXY-L-PROLIN UND N-METHYL-CIS-4-HYDROXY-L-PROLIN ZUR ANWENDUNG ALS THERAPEUTISCHER WIRKSTOFF INSBESONDERE FÜR DIE KREBSTHERAPIE**
COMBINATION OF CIS-4-HYDROXY-L-PROLINE AND N-METHYL-CIS-4-HYDROXY-L-PROLINE FOR USE AS A THERAPEUTIC AGENT, IN PARTICULAR IN CANCER TREATMENT
COMBINAISON DE CIS-4-HYDROXY-L-PROPOLINE ET DE N-METHYLE-CIS-4-HYDROXY-L-PROPOLINE S'UTILISANT COMME PRINCIPE ACTIF THERAPEUTIQUE, NOTAMMENT EN THERAPIE ANTICANCEREUSE

(30) Priorität: 11.03.1996 DE 19609454
(43) Veröffentlichungstag der Anmeldung: 06.05.1999
(73) Patentinhaber: Hoerrmann, Wilhelm, Dr., 82393 Iffeldorf (DE)
(72) Erfinder: Hoerrmann, Wilhelm, Dr., 82393 Iffeldorf (DE)
(74) Vertreter: Grättinger & Partner (GbR)
(86) Internationale Anmeldenummer: DE9700486
(87) Internationale Veröffentlichungsnummer: WO9733578

(56) Entgegenhaltungen:
- WO-A-86/07053
- DE-A- 3 538 619
- JOURNAL OF THE NATIONAL CANCER INSTITUTE , Bd. 75, Nr. 2, 1985, Seiten 353-359, XP002038153 KLOHS ET AL: "COLLAGEN-PRODUCTION INHIBITORS EVALUATED AS ANTITUMOR AGENTS"
- BIOLOGY OF REPRODUCTION, Bd. 33, Nr. 1, 1985, Seiten 213-227, XP002038154 THORNTON ET AL: "collagen and the proliferation and differentiation of rat ventral prostate epithelial cells"
- CANCER RESEARCH, Bd. 41, Nr. 7, 1981, Seiten 2855-2862, XP002038155 LEWKO ET AL: "SENSITIVITY OF N-NITROSOMETHYLUREA-INDUCED MAMMARY TUMORS TO CIS-HYDROXYPROLINE, AN INHIBITOR OF COLLAGEN PRODUCTION"

## Beschreibung

### Einleitung

Die Erfindung betrifft eine Arzneistoff-Kombination, die als wesentliche Komponenten cis-4-Hydroxy-L-prolin (A) und N-Methyl-cis-4-hydroxy-L-prolin (B), oder ihre pharmakologisch verträglichen Derivate enthält, zur Anwendung als therapeutischer Wirkstoff, insbesondere für die Krebstherapie des Menschen. Die Erfindung betrifft ferner Arzneimittelzusammensetzungen, die diese Kombination enthalten, und ihre Verwendung zur Herstellung von Arzneimitteln zur Behandlung von Krebs oder des hormonsensitiven Prostatakarzinoms.

### Technischer Hintergrund

Aus der DE-Patentschrift 35 38 619 ist die Verwendung von cis-4-Hydroxy-L-prolin bei der Behandlung von Karzinomen und verwandten Tumoren bekannt. Ferner ist aus der EP-B-0 223 850 die Verwendung von N-Methyl-cis- 4-hydroxyprolin und N-Methyl -trans-4-hydroxyprolin bei der Behandlung von Astrocytomzellen bekannt. In dieser Patentschrift werden diese Derivate von Hydroxyprolin und Prolin sowohl in verschiedener Konfiguration, OH- Positionierung und Alkylierung genannt. Ferner ist in Cancer Detection and Prevention, Vol. 11, Nr. ½, 1987, Seite 66, ein Abstract Nr. 2:046, W. Hoerrmann et al., "Differential effects of cis-4-hydroxy-L-proline (CHP) and methyl-cis-4-hydroxy-L-proline (mcHP) on tumour cells: Evidence for redifferentiation and cell growth inhibition", veröffentlicht worden.

### Die Erfindung

Es wurde nun überraschenderweise gefunden, daß die Kombination der Komponenten (A) und (B), d.h. cis-4-Hydroxy-L-prolin und N-Methyl-cis-4-hydroxy-L-prolin, eine wertvolle Arzneistoff-Kombination ist, die sich insbesondere zur Therapie von Tumoren eignet. In der Zellkultur von Tumorzellen kann diese antitumoröse Wirkung in besonders klarer Weise nachgewiesen werden, da sie zu einer signifikanten Hemmung der Zellproliferation führt. Die Tumorzellen werden dabei in einem üblichen Kulturmedium mit unterschiedlichen Konzentrationen von 1 bis 100 µg/ml der Kombination im Molverhältnis 1:1 über einen Zeitraum von 6 bis 10 Tagen behandelt. Zu bestimmten Zeiten wird die Zellzahl bestimmt. Die Abnahme der Zellzahl bzw. die Hemmung der Zellproliferation ist ein wichtiges Indiz für einen mit der Kombination therapierbaren Tumor.

Die erfindungsgemäße Kombination enthält die Komponenten (A) und (B) in der Regel in einem eine synergistische Wirkung erzeugenden Molverhältnis, vorzugsweise in einem Bereich von 10:1 bis 1:10 und besonders bevorzugt in einem Molverhältnis von 1:1. Beide Komponenten sind untoxisch mit einer akuten bzw. subakuten Toxizität von höher als 10.000 mg pro kg. Beide Komponenten erwiesen sich im Ames-Test als nicht mutagen.

Spezielle Beispiele für therapierbare Tumoren, deren Zellen bei der Behandlung mit der erfindungsgemäßen Kombination einer Hemmung der Zellproliferation zeigten, sind Nierenkarzinome, Harnblasenkarzinome, Prostatakarzinome, Mammakarzinome, Hirntumore und Fibrosarkome. Typische humane Tumorzellinien zum Testen der erfindungsgemäßen Kombination können von verschiedenen Kultursammlungen, z.B. der Zellkultursammlung des Deutschen Krebsforschungszentrums (DKFZ) in Heidelberg von der American Type Culture Collection (ATCC), Rockville, Maryland, U.S.A., und der European Collection of Cell Cultures (ECACC), Centre for Applied Microbiology and Research, Salisbury, Wiltshire, England, bezogen werden.

Die Beispiele erläutern die Erfindung. Sie sind nicht einschränkend aufzufassen.

### Beispiele: Versuche zur Hemmung der Zellproliferation

Für die Versuche zur Bestimmung der Hemmung der Zellproliferation mit der Kombination der Erfindung wurden folgende bekannte Zellinien humaner Herkunft verwendet. Diese Versuche wurden insofern nicht an in Nacktmäuse implantierte Tumoren durchgeführt, da die chemische Natur der Komponenten (A) und (B) eine Verstoffwechselung durch den tierischen Organismus möglich erscheinen läßt und damit die Gefahr falsch-negativer Resultate gegeben ist.
1. Nierenkarzinomzellen
   **KTCTL2**: Es handelt sich um eine Nierenkarzinomzellinie des DKFZ Heidelberg. KTCTL2-Zellen besitzen maligne Eigenschaften, bilden Tumore in Nacktmäusen, Kolonien im Weichgar und wachsen in Zellkultur stark adhärent auf der Unterlage.
   **SK Nep**: Diese Zellinie des DKFZ wurde ebenfalls aus einem Nierenzellkarzinom etabliert. DK Nep-Zellen bilden ebenso wie KTCTL2-Zellen Tumore in Nacktmäusen und Kolonien im Weichgar. Sie weisen aber morphologisch in Zellkultur einen höher malignen Phänotyp auf, da sie offenbar spontan kleine Aggregate bilden und fast unabhängig von der Adhärenz auf einer Unterlage ohne Kontakthemmung übereinanderwachsen.
   Diese zwei Zellinien wurden gewählt, da sie in vitro zunehmend maligne Eigenschaften aufweisen.
2. Harnblasenkarzinomzellen
   **RT-4**: Diese Zellinie stammt aus einem hochdifferenzierten papillären Harnblasenkarzinom (Grad1) eines Mannes. Diese Zellinie wächst stark adhäsionsabhängig.
   **J82**: Diese Zellinie stammt aus einem völlig verwilderten, gering differenzierten Harnblasenkarzinom (Grad 3).
3. Prostatakarzinomzellen
   **PC-3**: Eine Zellinie aus einem progredienten, hormonresistenten Prostatakarzinom. Sie ist hochgradig tumorig in Nacktmäusen und bildet Kolonien in Weichagar.
   **LNCaP**: Zellinie aus einer Lymphknotenmetastase eines hormonsensitiven, mittelgradig differenzierten Prostatakarzinoms.
   - Mammakarzinomzellen:: Es wurde die Zellinie MCF 7 verwendet; sie ist oestrogensensitiv.
   - Fibrosarkomzellen:: Es wurde die Zellinie HT 1080 verwendet.
   - Hirntumorzellen:: Es wurden die Zellinien HTZ-122 und HTZ-17 verwendet. Bei der Zellinie HTZ-122 handelt es sich um ein niedrig malignes Astrocytom (WHO Grad I), bei der Zellinie HTZ-17 um einen hochmalignen astrocytären Tumor (Glioblastoma multiforme, WHO Grad IV).

Es wurden mit diesen Zellinien folgende Behandlungsversuche durchgeführt:
1. Es wurde die Substanz (A) cis-4-Hydroxy-L-prolin in verschiedenen Konzentrationen allein eingesetzt.
2. Es wurde die Substanz (B) N-Methyl-cis-4-hydroxy-L-prolin in verschiedenen Konzentrationen allein eingesetzt.
3. Es wurde die Kombination (K) aus (A) und (B) im Molverhältnis 1:1 in verschiedenen Konzentrationen eingesetzt.

Das Ergebnis aller dieser Versuche ist den beigefügten graphischen Darstellungen zu entnehmen, aus denen auch die jeweiligen Zellzahlen, die Versuchsdauer in Tagen und die verwendeten Konzentrationen ersichtlich sind.

Diese graphischen Darstellungen werden nachfolgend erläutert:

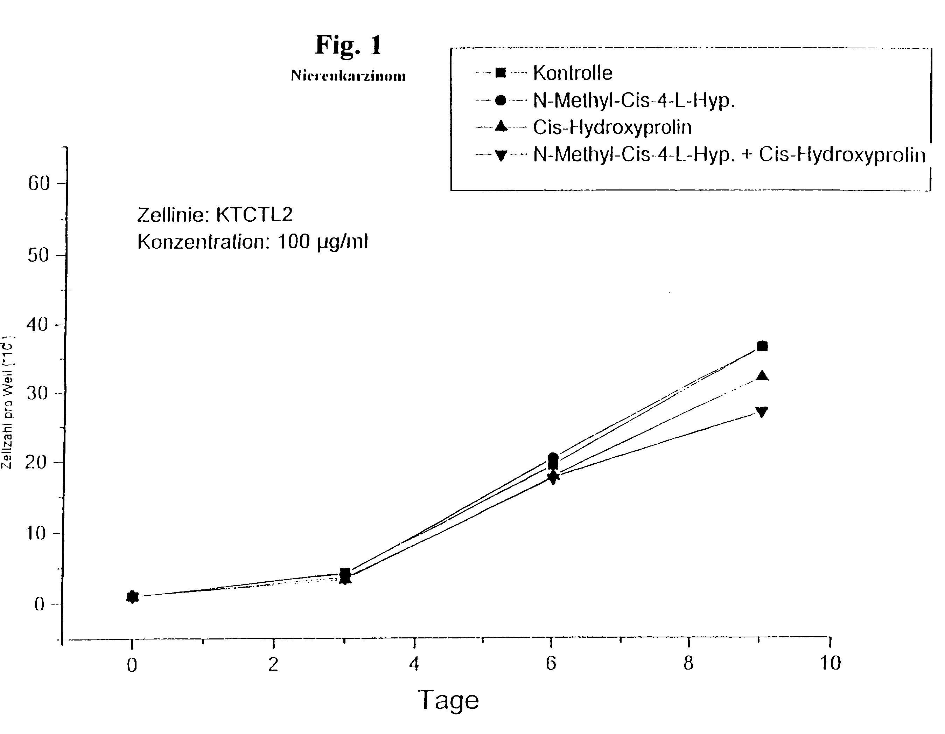

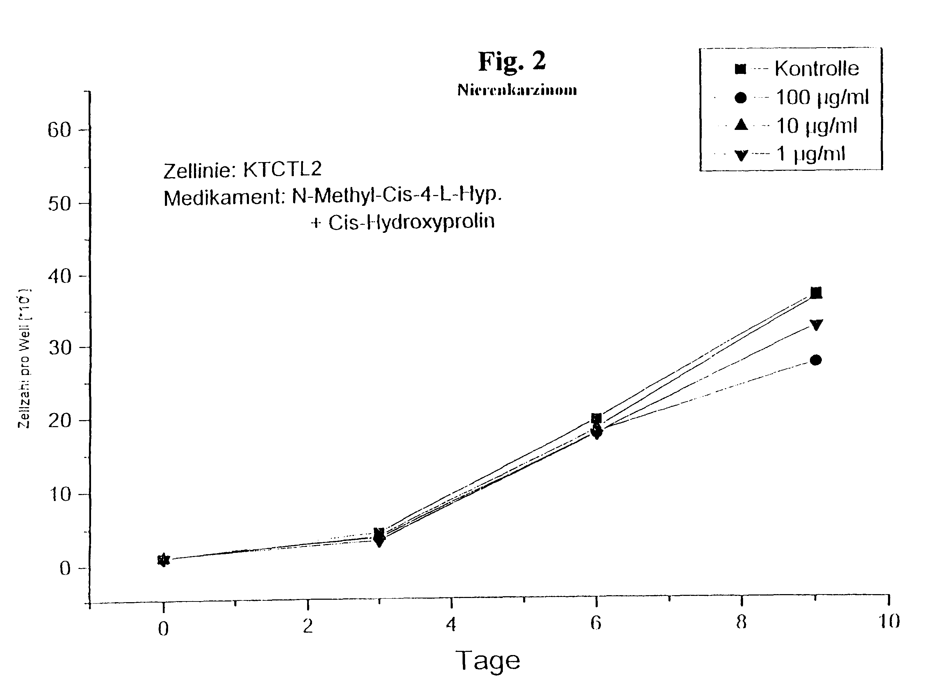

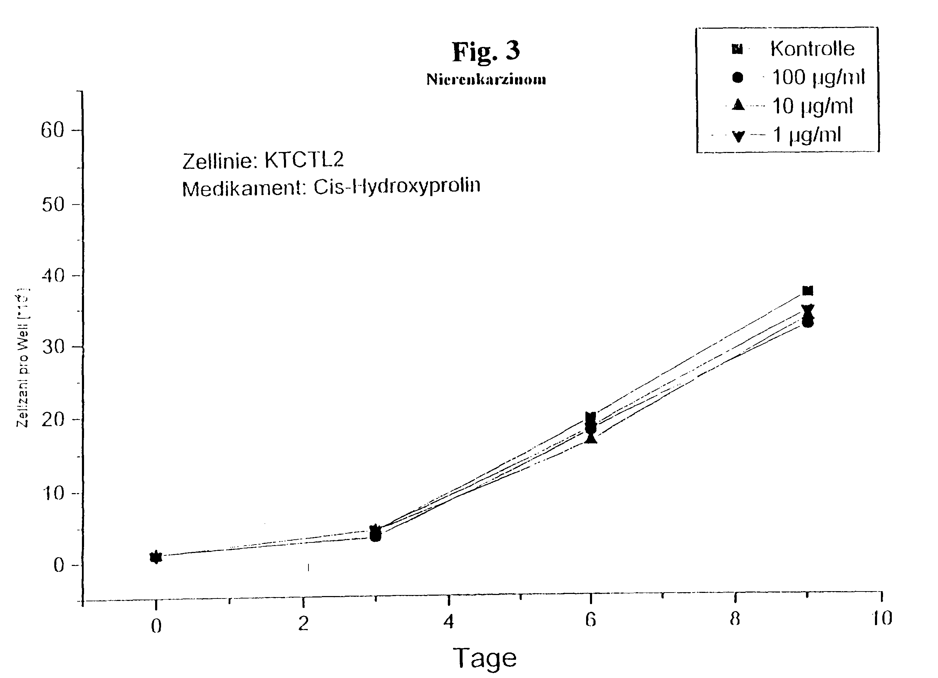

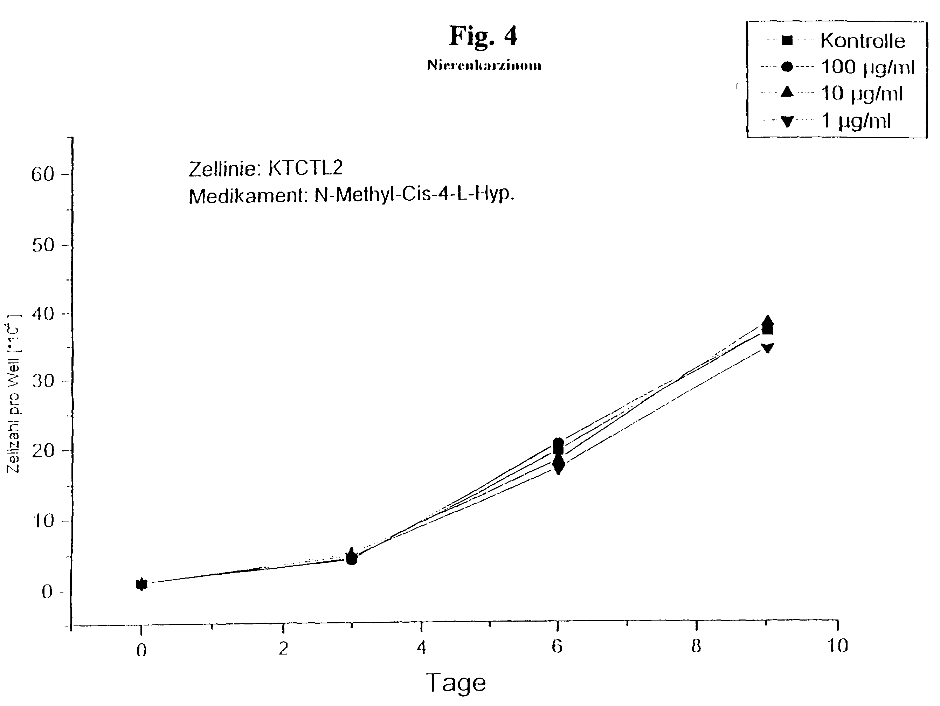

Bei der Nierenkarzinom-Zellinie KTCTL2 haben die Einzelsubstanzen (A) und (B) keine Wirkung. Dagegen führt die Kombination (K) aus beiden Substanzen zu einer signifikanten und dosisabhängigen Wachstumshemmung von ca. 25%. Siehe graphische Darstellung Figuren 1 bis 4. Die Kombination zeigt somit eine echte kooperative (synergistische) Hemmung der Zellproliferation bei KTCTL2 Zellen, die allein durch keine der beiden Substanzen erzielt werden kann.

Im Gegensatz dazu fanden sich bei SK Nep, die einen besonders malignen Phänotyp aufweist, für (A), (B) und (K) keine Wirkung.

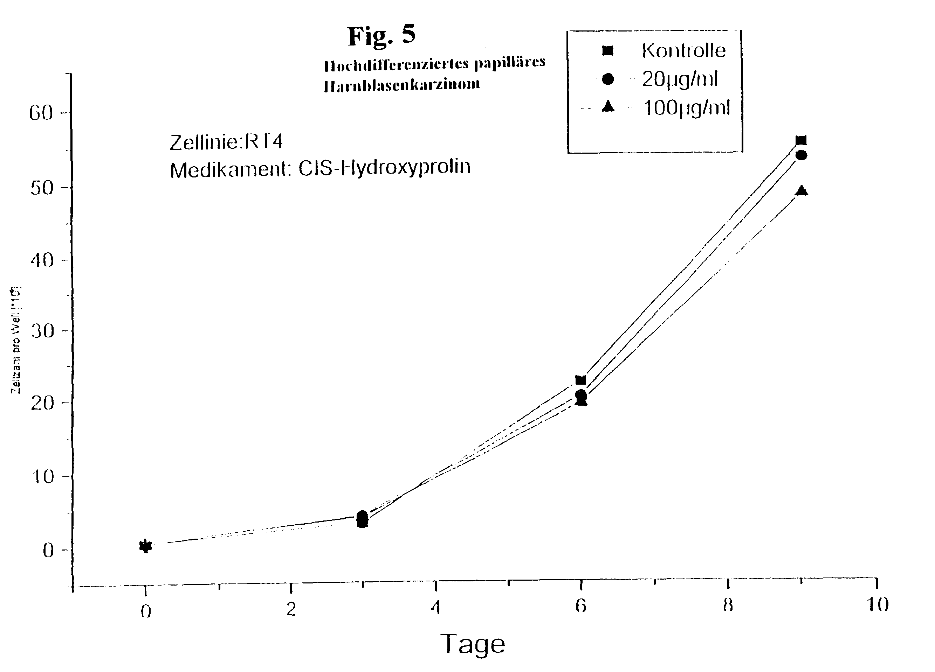

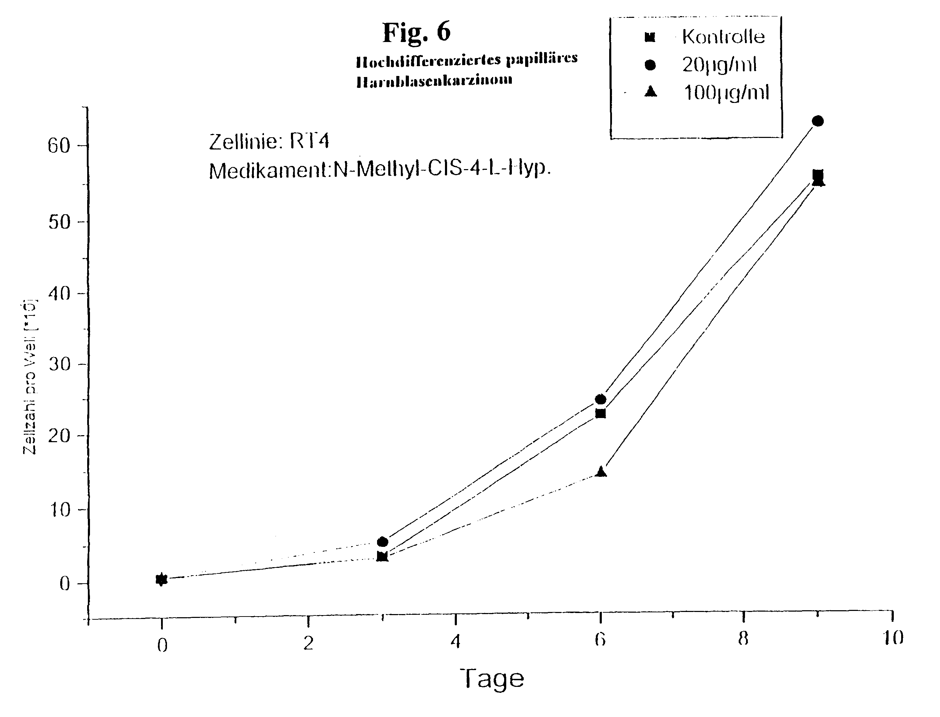

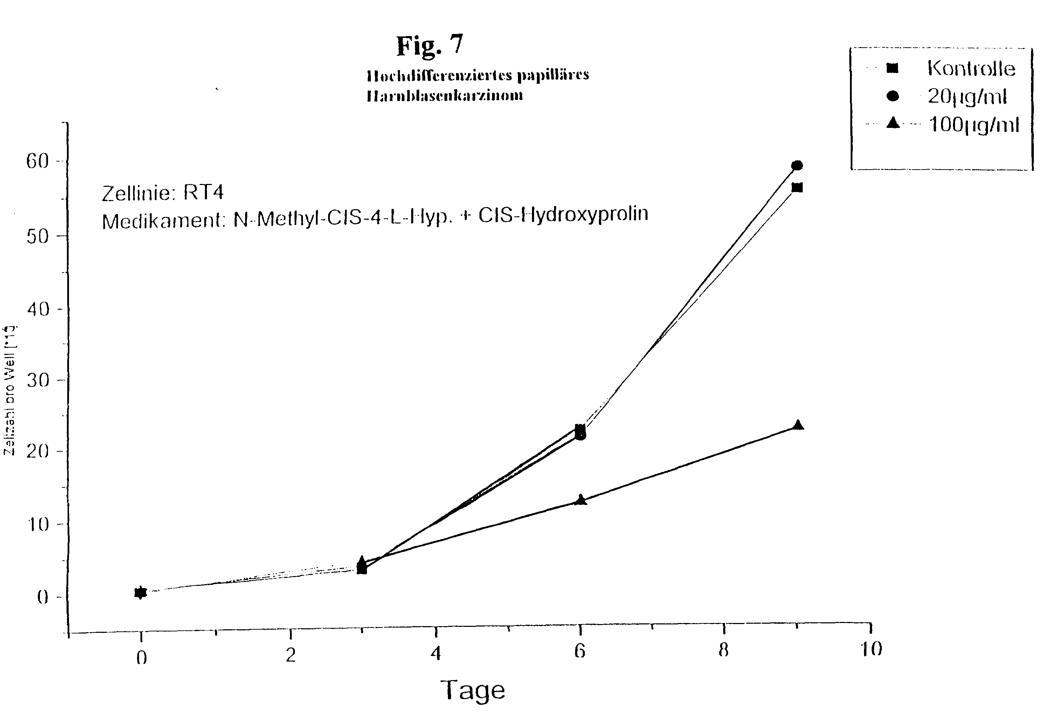

Beeindruckend ist der sehr starke kooperative (synergistische) Effekt der Kombination (K) auf die Zellinie RT-4 aus einem papillären Harnblasenkarzinom eines Mannes. In der Konzentration von 100 µg/ml zeigen die Einzelsubstanzen praktisch keinen Effekt, in der Kombination (K) dagegen eine drastische Hemmung der Zellproliferation. Siehe Figuren 5 bis 7.

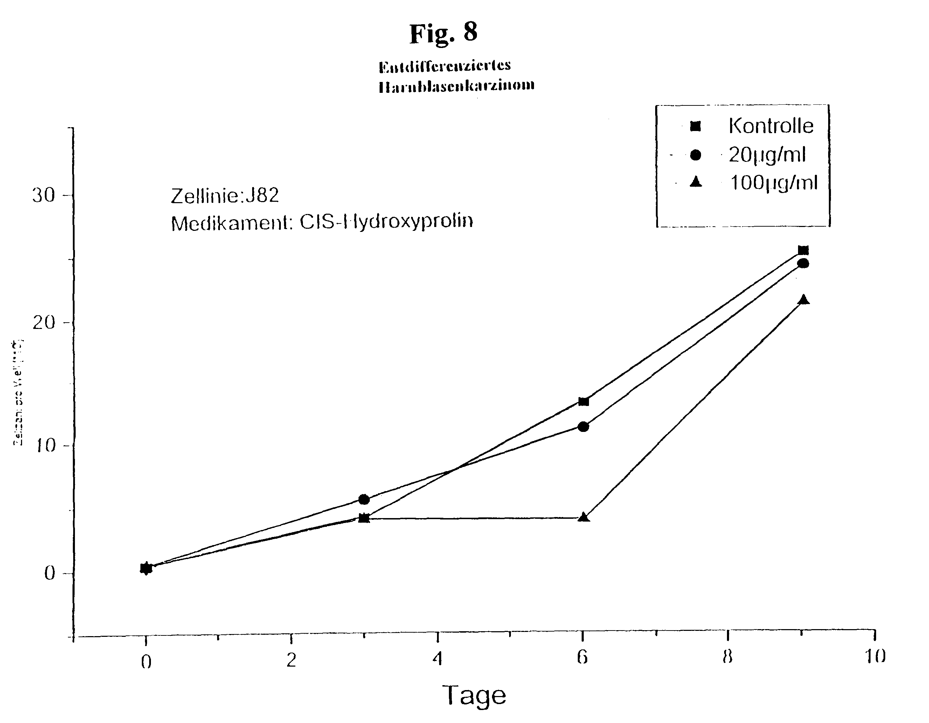

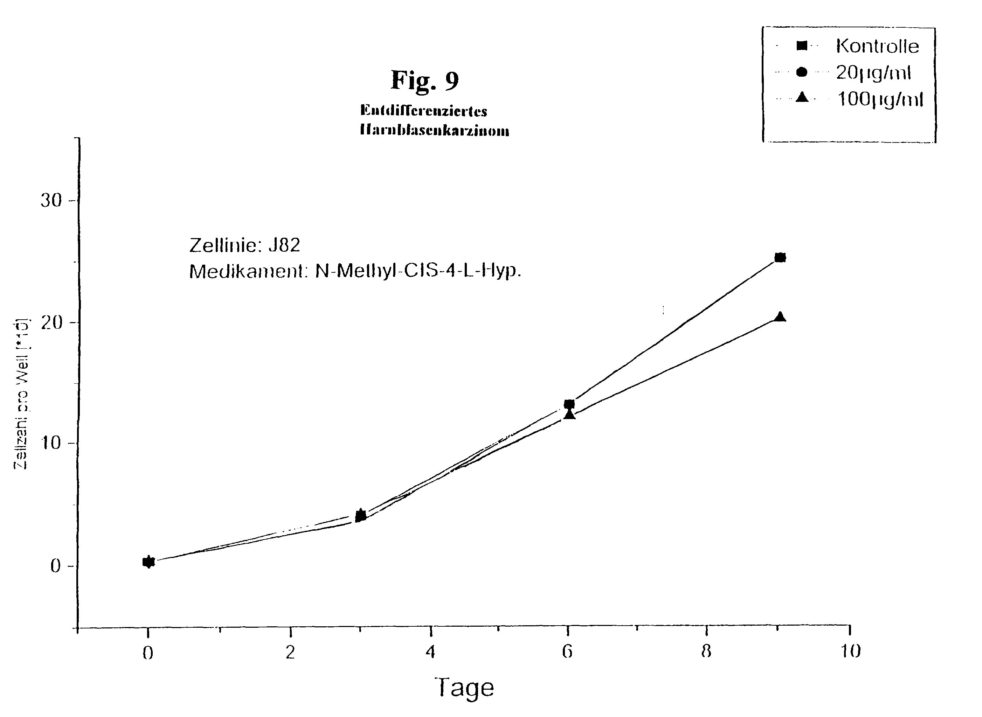

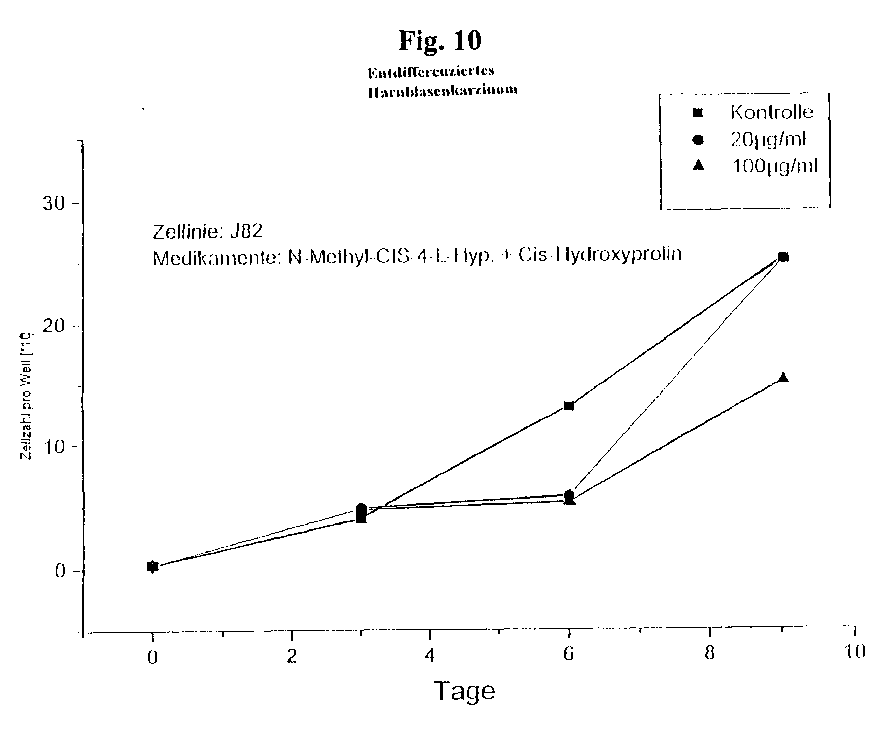

Bei dem völlig verwilderten, gering differenzierten Harnblasenkarzinom J 82 zeigten die Einzelsubstanzen (A) und (B) keine, die Kombination (K) eine mäßige Wirkung. Siehe Figuren 8 bis 10.

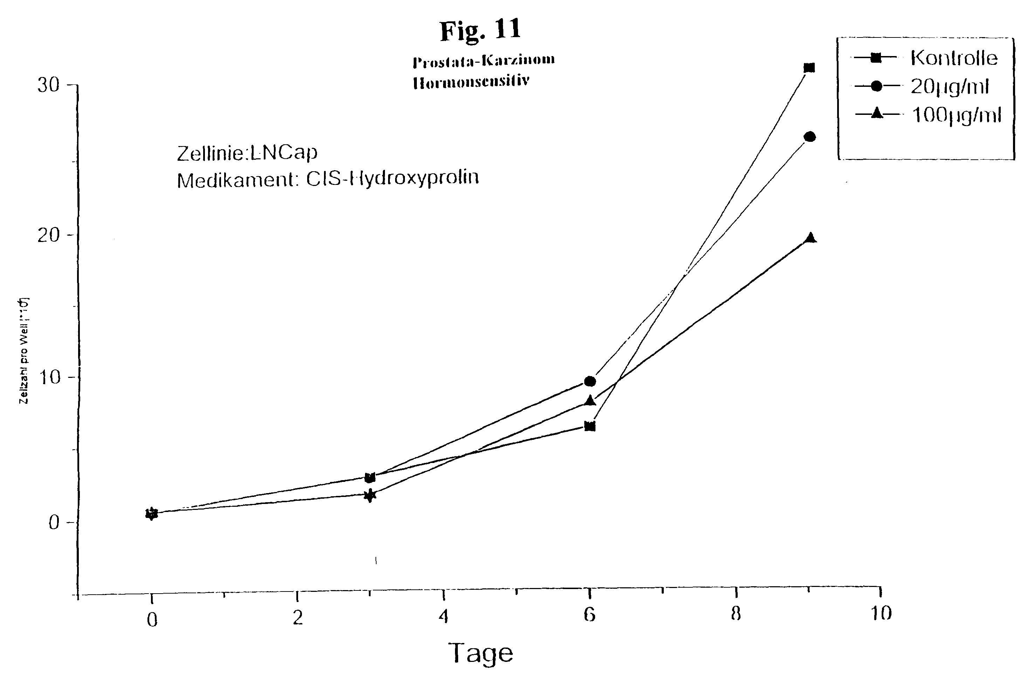

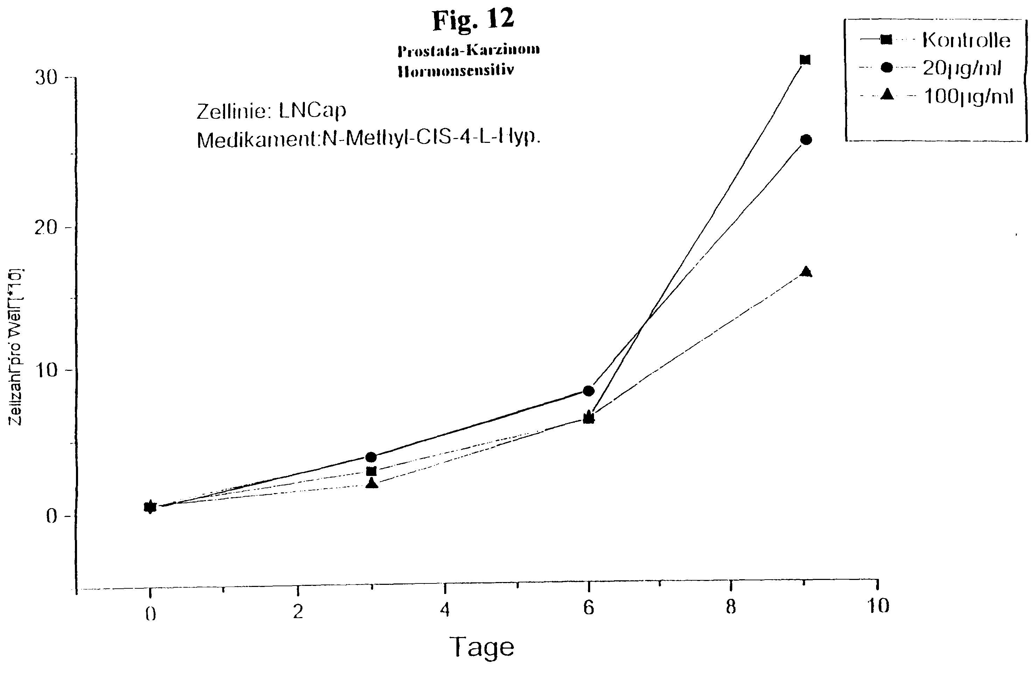

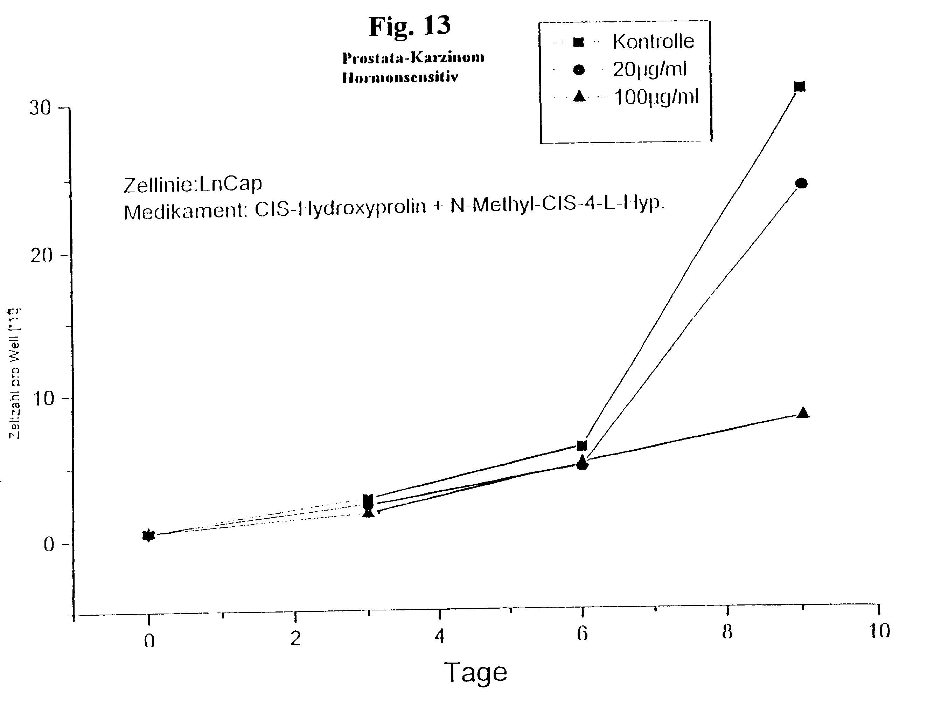

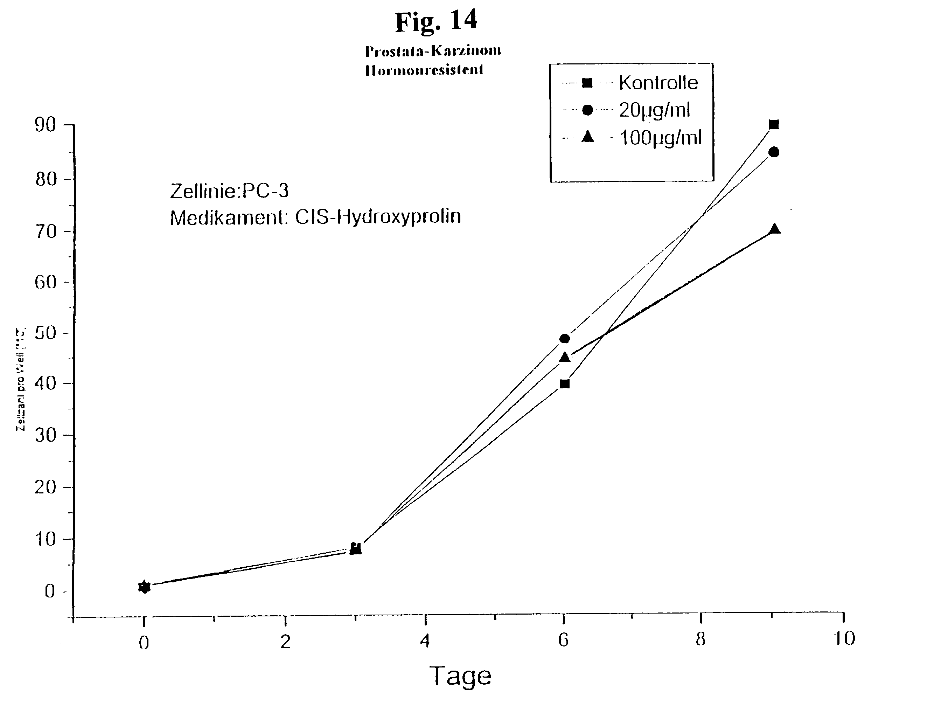

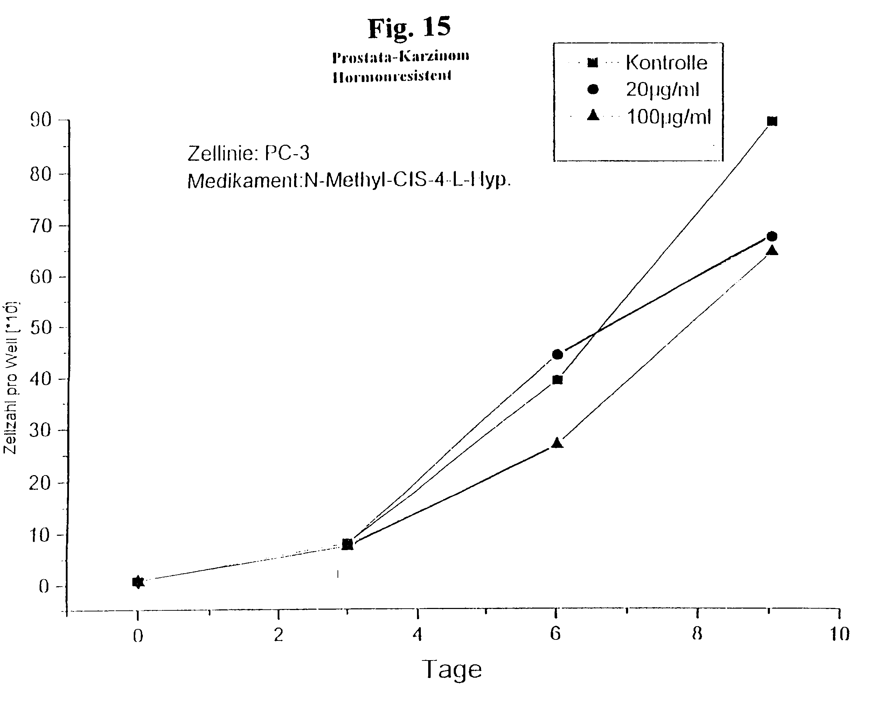

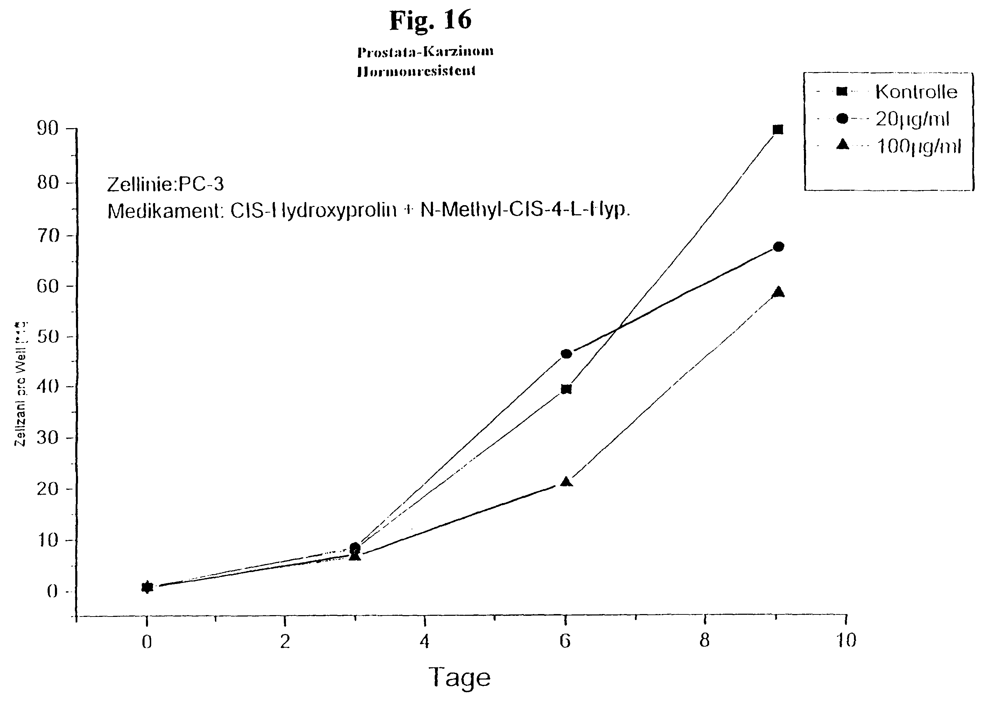

Bei der hormonsensitiven Prostatakarzinom-Zellinie LNCaP findet sich eine signifikante Hemmung der Zellproliferation sowohl für die Einzelsubstanz (A) als auch (B), noch stärker aber für die Kombination (K). Siehe Figuren 11 bis 13.

Dagegen findet sich bei der hormonresistenten Prostatakarzinom-Zellinie PC 3 sowohl für die Einzelsubstanz (A) und (B) als auch für die Kombination (K) nur eine schwache Wirkung. Siehe 14 bis 16.

Das unterschiedliche Verhalten dieser beiden Prostatakarzinom-Zellinien beweist die hohe Spezifität der eingesetzten Verbindungen.

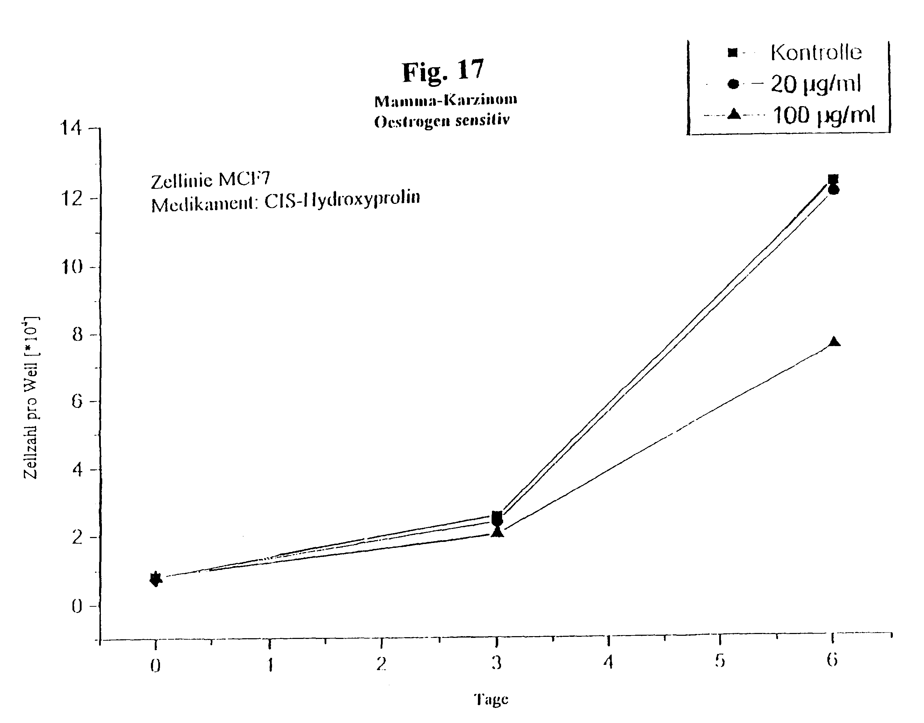

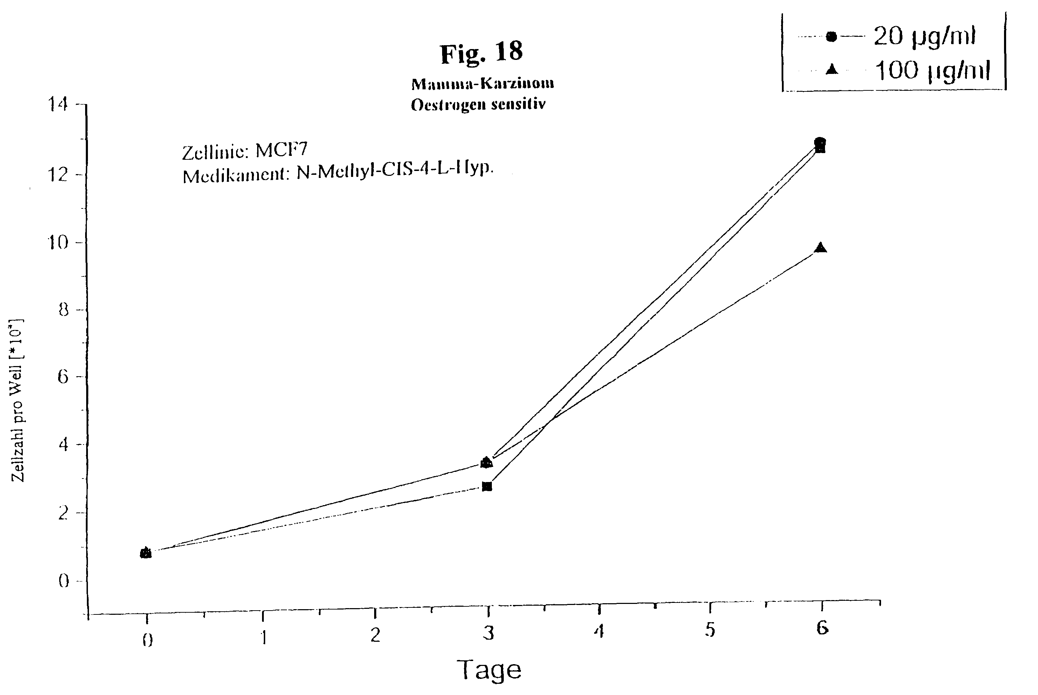

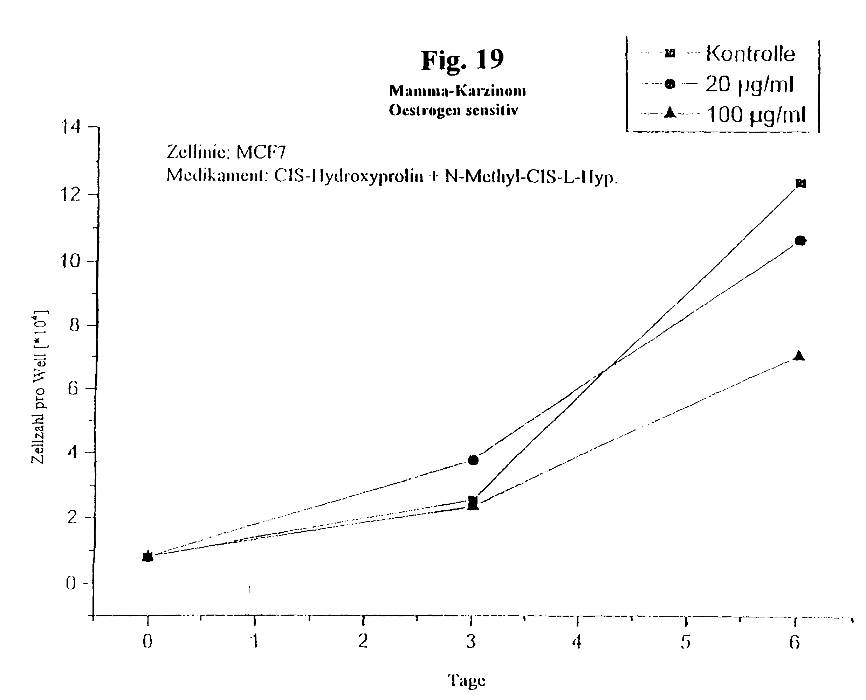

Bei der oestrogensensitiven Mammakarzinom-Zellinie MCF 7 zeigen sowohl die Einzelsubstanzen (A) und (B) als auch die Kombination (K) eine Hemmung bis zu 50%, aber nur für die Kombination konnte gezeigt werden, daß dieser Effekt dosisabhängig ist. Siehe Figuren 17 bis 19.

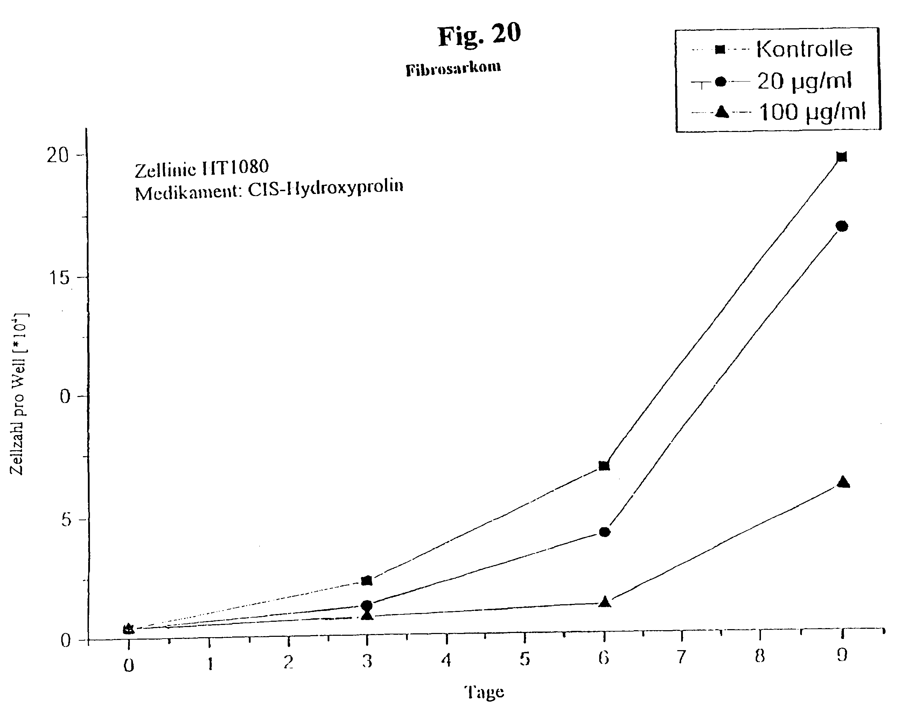

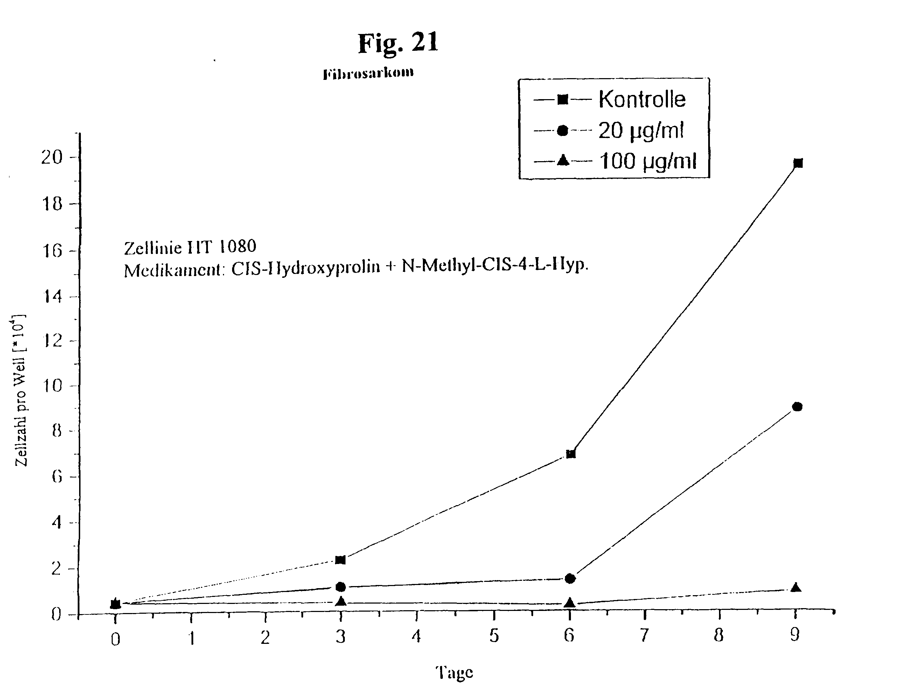

Bei der Fibrosarkom-Zellinie HT 1080 zeigt bereits die Einzelsubstanz (A) eine starke Wirkung, für (B) war die Kurve nicht auswertbar, die Kombination (K) zeigt eine hervorragende Wirkung mit einer dosisabhängigen Hemmung der Zellproliferation von mehr als 90%. Siehe Figuren 20 bis 21.

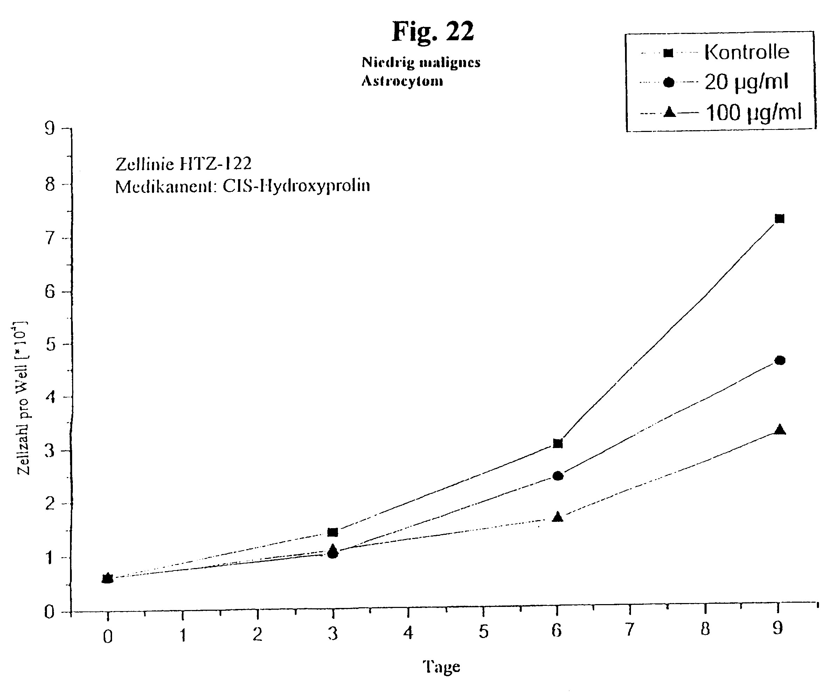

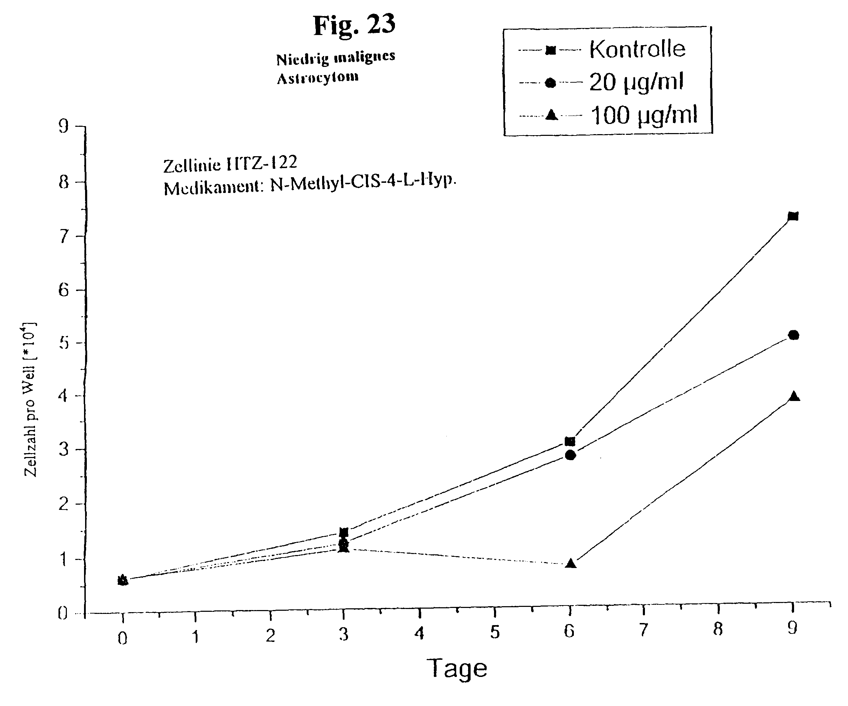

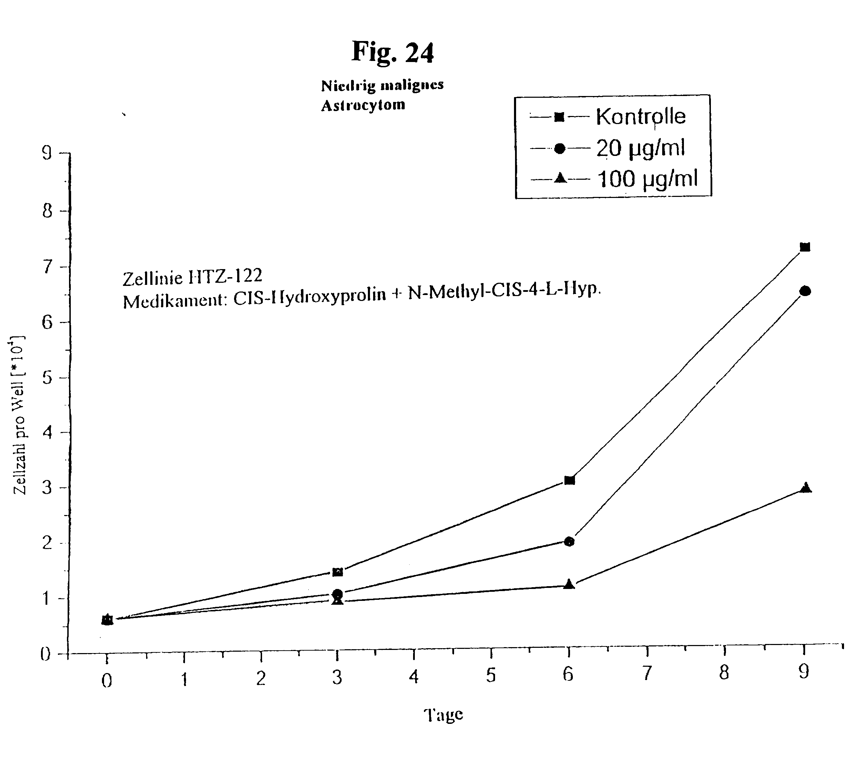

Bei der Hirntumor-Zellinie HTZ 122 sind die Einzelsubstanzen (A) und (B) sowie die Kombination (K) wirksam, die Kombination (K) aber am stärksten mit einer Hemmung um ca. 66%. Dabei ist dieser Effekt dosisabhängig. Siehe Figuren 22 bis 24.

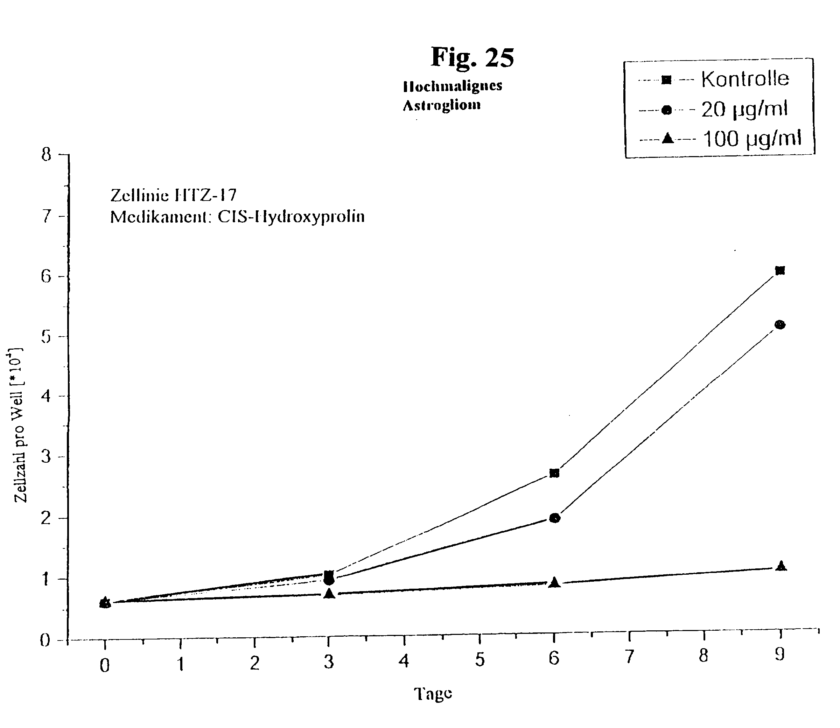

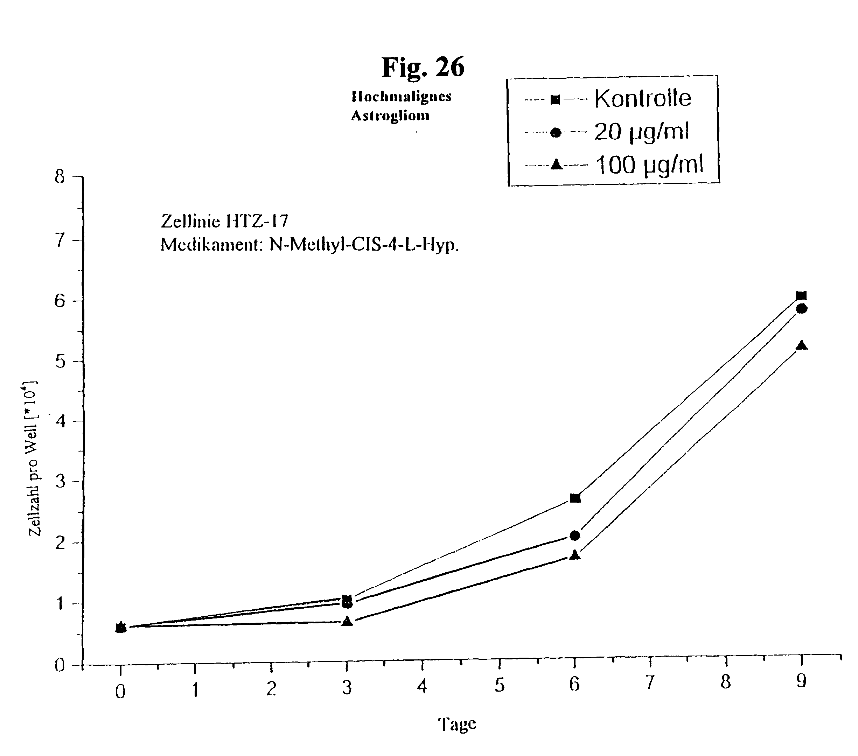

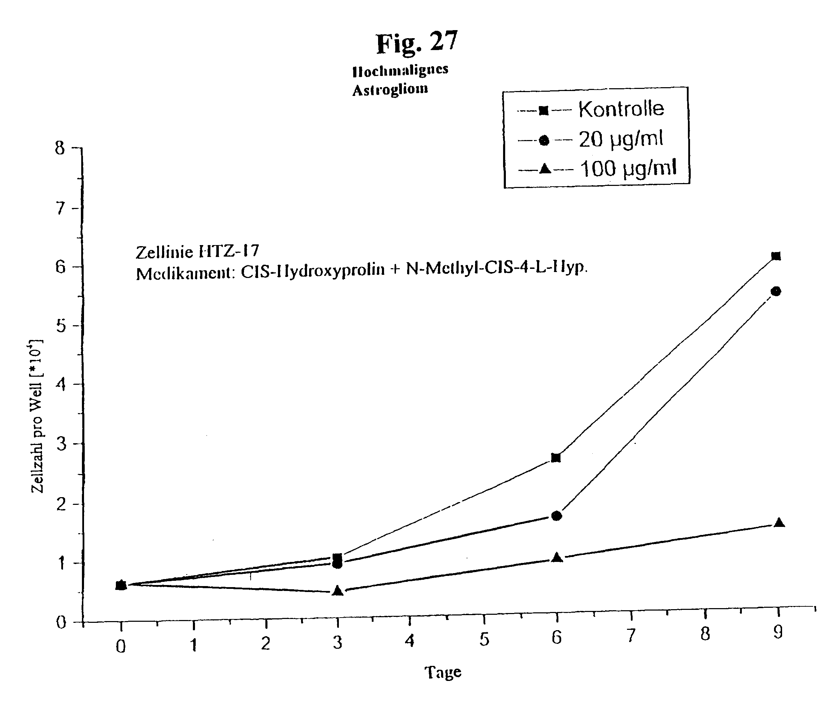

Bei der hochmalignen Hirntumor-Zellinie HTZ 17 ist bereits die Einzelsubstanz (A) hervorragend wirksam, die Einzelsubstanz (B) nur sehr gering wirksam, die Kombination (K) erreicht eine Hemmung von ca. 75%. Siehe Figuren 25 bis 27.

### Arzneimittel und Therapie

Arzneimittelzusammensetzungen, die eine Kombination der Erfindung enthalten, können nach an sich bekannten Methoden hergestellt werden. Verwiesen wird auf das Buch "Remington's Pharmaceutical Sciences", 18. Auflage, 1990, Mack Publishing Company, Easton, Pennsylvania, U.S.A., insbesondere Teil 6, Pharmaceutical Preparations and Their Manufacture. Die Einzelsubstanzen sind durch chemische Synthese oder im Handel erhältlich. Die Zusammensetzungen können zur oralen oder parenteralen, insbesondere intravenösen Applikation mit üblichen Hilfsstoffen formuliert werden. Zur Behandlung des Harnblasenkarzinoms kommt auch die intravesikale (transurethrale) Applikation in Frage. In diesem Fall wird die Kombination in Form einer verdünnten wäßrigen Lösung verabfolgt.

Die Dosis hängt von der Schwere der Erkrankung ab. Die Kombination der Erfindung kann in einer Einzeldosis von 0,01 bis 0,1 g pro kg Körpergewicht einmal oder mehrmals täglich appliziert werden.

Die erfindungsgemäßen Arzneimittel können die Komponenten (A) und (B) auch in Form von pharmakologisch verträglichen Abkömmlingen oder Vorläufern enthalten. Hier sind zu nennen: Alkalisalze, Erdalkalisalze, Säureadditionssalze, Ester, Amide, Säuredamide und Äther der genannten Verbindungen, Dehydroprolin und N-Alkyl- bzw. N-Methyl-Derivate des Dehydroprolins und ihre entsprechenden Keto-Verbindungen.

## Patentansprüche

1. Kombination aus cis-4-Hydroxy-L-prolin als Komponente (A) und N-Methyl-cis-4-hydroxy-L-prolin als Komponente (B), oder ihre pharmakologisch verträglichen Derivate in einem eine synergistische Wirkung erzeugenden Molverhältnis zur Anwendung als therapeutischer Wirkstoff.

2. Kombination nach Anspruch 1 im Molverhältnis der Komponenten (A) und (B) von 10:1 bis 1:10.

3. Kombination nach Anspruch 1 im Molverhältnis der Komponenten (A) und (B) von 1:1.

4. Kombination nach einem der Ansprüche 1 bis 3 zur Anwendung bei der Therapie von Tumoren.

5. Kombination nach Anspruch 4 zur Anwendung bei der Therapie des Harnblasenkarzinoms.

6. Kombination nach Anspruch 4 zur Anwendung bei der Therapie des hormonsensitiven Prostatakarzinoms.

7. Kombination nach Anspruch 4 zur Anwendung bei der Therapie des Nierenkarzinoms.

8. Kombination nach Anspruch 4 zur Anwendung bei der Therapie des hormonsensitiven Mammakarzinoms.

9. Kombination nach Anspruch 4 zur Anwendung bei der Therapie des Fibrosarkoms.

10. Kombination nach Anspruch 4 zur Anwendung bei der Therapie von Hirntumoren.

11. Arzneimittel, enthaltend eine Kombination aus cis-4-Hydroxy-L-prolin als Komponente (A) und N-Methyl-cis-4-hydroxy-L-prolin als Komponente (B), oder ihre pharmakologisch verträglichen Derivate in einem bei der Hemmung der Zellproliferation von Tumorzellen eine synergistische Wirkung erzeugenden Molverhältnis.

12. Arzneimittel nach Anspruch 10, enthaltend die Komponenten (A) und (B) im Molverhältnis 10:1 bis 1:10.

13. Arzneimittel nach Anspruch 10, enthaltend die Komponenten (A) und (B) im Molverhältnis 1:1.

14. Arzneimittel nach einem der Ansprüche 11 bis 13 zur Anwendung bei der Therapie von menschlichen Tumoren.

15. Arzneimittel nach Anspruch 14 zur Anwendung bei der Therapie des Harnblasenkarzinoms.

16. Arzneimittel nach Anspruch 14 zur Anwendung bei der Therapie des hormonsensitiven Prostatakarzinoms.

17. Arzneimittel nach Anspruch 14 zur Anwendung bei der Therapie des Nierenkarzinoms.

18. Arzneimittel nach Anspruch 13 zur Anwendung bei der Therapie des hormonsensitiven Mammakarzinoms.

19. Arzneimittel nach Anspruch 14 zur Anwendung bei der Therapie des Fibrosarkoms.

20. Arzneimittel nach Anspruch 14 zur Anwendung bei der Therapie von Hirntumoren.

21. Verwendung einer Kombination aus cis-4-Hydroxy-L-prolin als Komponente (A) und N-Methyl-cis-4-hydroxy-L-prolin als Komponente (B), oder ihrer pharmakologisch verträglichen Derivate in einem eine synergistische Wirkung erzeugenden Molverhältnis zur Herstellung einer Arzneimittelzusammensetzung für die Therapie von Tumoren.

22. Verwendung nach Anspruch 21 zur Herstellung einer Arzneimittelzusammensetzung für die Therapie des Harnblasenkarzinoms.

23. Verwendung nach Anspruch 21 zur Herstellung einer Arzneimittelzusammensetzung für die Therapie des hormonsensitiven Prostatakarzinoms.

24. Verwendung nach Anspruch 21 zur Herstellung einer Arzneimittelzusammensetzung für die Therapie des Nierenkarzinoms.

25. Verwendung nach Anspruch 21 zur Herstellung einer Arzneimittelzusammensetzung für die Therapie des hormonsensitiven Mammakarzinoms.

26. Verwendung nach Anspruch 21 zur Herstellung einer Arzneimittelzusammensetzung für die Therapie des Fibrosarkoms.

27. Verwendung nach Anspruch 21 zur Herstellung einer Arzneimittelzusammensetzung für die Therapie von Hirntumoren.

28. Verwendung nach einem der Ansprüche 21 bis 27, wobei das Molverhältnis der Komponenten (A) und (B) 10:1 bis 1:10, insbesondere 1:1 beträgt.

## Claims

1. A combination of cis-4-hydroxy-L-proline as component (A) and N-methyl-cis-4-hydroxy-L-proline as component (B), or their pharmacologically well-tolerated derivatives, in a molar ratio producing a synergistic effect for use as a therapeutic agent.

2. The combination according to claim 1 in a molar ratio of the components (A) and (B) of 10:1 to 1:10.

3. The combination according to claim 1 in a molar ratio of the components (A) and (B) of 1:1.

4. The combination according to one of the claims 1 through 3 for use in the therapy of tumors.

5. The combination according to claim 4 for use in the therapy of bladder carcinoma.

6. The combination according to claim 4 for use in the therapy of hormone-sensitive prostate carcinoma.

7. The combination according to claim 4 for use in the therapy of kidney carcinoma.

8. The combination according to claim 4 for use in the therapy of hormone-sensitive mamma carcinoma.

9. The combination according to claim 4 for use in the therapy of fibrosarcoma.

10. The combination according to claim 4 for use in the therapy of brain tumors.

11. A medication, comprising a combination of cis-4-hydroxy-L-proline as component (A) and N-methyl-cis-4-hydroxy-L-proline as component (B), or their pharmacologically well-tolerated derivatives, in a molar ratio producing a synergistic effect in the inhibition of cell proliferation of tumor cells.

12. The medication according to claim 10, comprising the components (A) and (B) in a molar ratio of 10:1 to 1:10.

13. The medication according to claim 10, comprising the components (A) and (B) in a molar ratio of 1:1.

14. The medication according to one of the claims 11 through 13 for use in the therapy of human tumors.

15. The medication according to claim 14 for use in the therapy of bladder carcinoma.

16. The medication according to claim 14 for use in the therapy of hormone-sensitive prostate carcinoma.

17. The medication according to claim 14 for use in the therapy of kidney carcinoma.

18. The medication according to claim 13 for use in the therapy of hormone-sensitive mamma carcinoma.

19. The medication according to claim 14 for use in the therapy of fibrosarcoma.

20. The medication according to claim 14 for use in the therapy of brain tumors.

21. A use of a combination of cis-4-hydroxy-L-proline as component (A) and N-methyl-cis-4-hydroxy-L-proline as component (B), or their pharmacologically well-tolerated derivatives, in a molar ratio producing a synergistic effect for preparing a medication composition for the therapy of tumors.

22. The use according to claim 21 for preparing a medication composition for the therapy of bladder carcinoma.

23. The use according to claim 21 for preparing a medication composition for the therapy of hormone-sensitive prostate carcinoma.

24. The use according to claim 21 for preparing a medication composition for the therapy of kidney carcinoma.

25. The use according to claim 21 for preparing a medication composition for the therapy of hormone-sensitive mamma carcinoma.

26. The use according to claim 21 for preparing a medication composition for the therapy of fibrosarcoma.

27. The use according to claim 21 for preparing a medication composition for the therapy of brain tumors.

28. The use according to one of the claims 21 through 27, wherein the molar ratio of the components (A) and (B) is 10:1 to 1:10, in particular 1:1.

## Revendications

1. Combinaison de cis-4-hydroxy-L-propoline comme composant (A) et de N-méthyle-cis-4-hydroxy-L-propoline comme composant (B), ou de leurs dérivés pharmacologiquement compatibles, dans un rapport molaire produisant une synergie, en vue d'une application comme principe actif thérapeutique.

2. Combinaison selon la revendication 1, dans le rapport molaire des composants (A) et (B) de 10:1 à 1:10.

3. Combinaison selon la revendication 1, dans le rapport molaire des composants (A) et (B) de 1:1.

4. Combinaison selon l'une des revendications 1 à 3, pour application à la thérapie des tumeurs.

5. Combinaison selon la revendication 4, pour application à la thérapie du carcinome de la vessie.

6. Combinaison selon la revendication 4, pour application à la thérapie du carcinome de la prostate hormonosensible.

7. Combinaison selon la revendication 4, pour application à la thérapie du carcinome rénal.

8. Combinaison selon la revendication 4, pour application à la thérapie du carcinome mammaire hormonosensible.

9. Combinaison selon la revendication 4, pour application à la thérapie du fibrosarcome.

10. Combinaison selon la revendication 4, pour application à la thérapie de tumeurs au cerveau.

11. Médicament contenant une combinaison de cis-4-hydroxy-L-propoline comme composant (A) et de N-méthyle-cis-4-hydroxy-L-propoline comme composant (B), ou de leurs dérivés pharmacologiquement compatibles, dans un rapport molaire produisant une synergie lors de l'inhibition de la prolifération de cellules tumorales.

12. Médicament selon la revendication 10, contenant les composants (A) et (B) dans le rapport molaire de 10:1 à 1:10.

13. Médicament selon la revendication 10, contenant les composants (A) et (B) dans le rapport molaire de 1:1.

14. Médicament selon l'une des revendications 11 à 13, pour application à la thérapie de tumeurs humaines.

15. Médicament selon la revendication 14, pour application à la thérapie du carcinome de la vessie.

16. Médicament selon la revendication 14, pour application à la thérapie du carcinome de la prostate hormonosensible.

17. Médicament selon la revendication 14, pour application à la thérapie du carcinome rénal.

18. Médicament selon la revendication 13, pour application à la thérapie du carcinome mammaire hormonosensible.

19. Médicament selon la revendication 14, pour application à la thérapie du fibrosarcome.

20. Médicament selon la revendication 14, pour application à la thérapie de tumeurs au cerveau.

21. Utilisation d'une combinaison de cis-4-hydroxy-L-propoline comme composant (A) et de N-méthyle-cis-4-hydroxy-L-propoline comme composant (B), ou de leurs dérivés pharmacologiquement compatibles, dans un rapport molaire produisant une synergie, en vue de la préparation d'une composition médicamenteuse pour la thérapie de tumeurs.

22. Utilisation selon la revendication 21, en vue de la préparation d'une composition médicamenteuse pour la thérapie du carcinome de la vessie.

23. Utilisation selon la revendication 21, en vue de la préparation d'une composition médicamenteuse pour la thérapie du carcinome de la prostate hormonosensible.

24. Utilisation selon la revendication 21, en vue de la préparation d'une composition médicamenteuse pour la thérapie du carcinome rénal.

25. Utilisation selon la revendication 21, en vue de la préparation d'une composition médicamenteuse pour la thérapie du carcinome mammaire hormonosensible.

26. Utilisation selon la revendication 21, en vue de la préparation d'une composition médicamenteuse pour la thérapie du fibrosarcome.

27. Utilisation selon la revendication 21, en vue de la préparation d'une composition médicamenteuse pour la thérapie de tumeurs au cerveau.

28. Utilisation selon l'une des revendications 21 à 27, suivant laquelle le rapport molaire des composants (A) et (B) est de 10:1 à 1:10, en particulier de 1:1.
